# EUROPEAN PATENT APPLICATION

(11) **EP 0 664 293 A1**
(43) Date of publication of application: **26.07.1995**
(21) Application number: 94203482.8
(22) Date of filing: 30.11.1994
(51) Int. Cl.: C07D 451/02, C07D 487/08, A61K 31/40, A61K 31/46

(54) **2-Phenyl-7-azabicycloheptanes and 6-phenyl-8-azabicyclo**

(30) Priority: 24.12.1993 EP 93203672
(71) Applicant: DUPHAR INTERNATIONAL RESEARCH B.V, NL-1380 AC Weesp (NL)
(72) Inventor: Scheeren, Johannes, Octrooibureau Zoan B.V., NL-1380 AC Weesp (NL); Aben, René W.M., Octrooibureau Zoan B.V., NL-1380 AC Weesp (NL); Kruse, Cornelis, Octrooibureau Zoan B.V., NL-1380 AC Weesp (NL)
(74) Representative: Muis, Maarten

(57) **Abstract**

The invention relates to a group of novel 2-phenyl-7-azabicyclo [2,2,1] heptane and 6-phenyl-8-azabicyclo [3,2,1] octane derivatives having central nervous system, in particular analgesic activity, and to a method for the preparation of these compounds.

The compounds have the general formula (1) 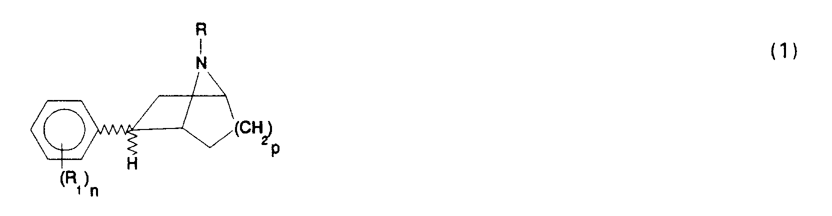
wherein R is hydrogen or alkyl (1-3C), R₁ is alkyl (1-3C), alkoxy (1-3C), halogen, hydroxy or esterified hydroxy, amino or acetylated amino, or methylenedioxy, n has the value 0-3, and p is 1 or 2.

## Description

The invention relates to a group of novel 2-phenyl-7-azabicycloheptanes and 6-phenyl-8-azabicyclooctanes, and to a process for the preparation thereof.

It is known that exo-2-(2-chloro-5-pyridyl)-7-azabicyclo [2,2,1] heptane, also known as epibatidine, is a potent non-opioid analgesic. Originally epibatidine was isolated from skins of the Ecuadoran poison frog. However, a number of routes for the synthesis of epibatidine have been published recently.

Further it is known from BPS.1,577,614 that a group of 6-phenyl-8-azabicyclo [3,2,1] octanes and - octenes also have interesting pharmacological properties, in particular central nervous system, cardiovascular and analgesic activity. All of the octane derivatives described in BPS.1,577,614 have an optionally esterified hydroxy group at carbon atom 6 of the azabicyclo ring system.

It has now been found that compounds of the formula (1) 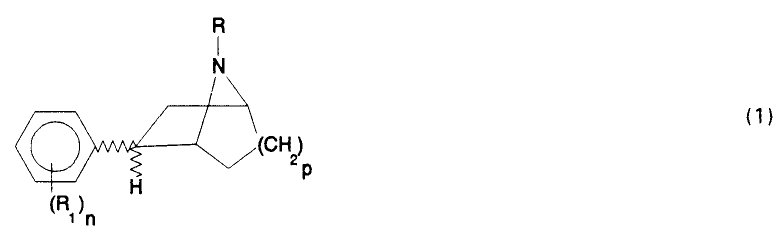
wherein R is hydrogen or alkyl (1-3 C), R₁ is alkyl (1-3 C), alkoxy (1-3 C),halogen, hydroxy or esterified hydroxy, amino or acetylated amino, or methylenedioxy, n has the value 0-3, and p is 1 or 2 and acid addition salts thereof have strong and interesting activity on the central nervous system, e.g. analgesic activity.

The presence of this analgesic activity in the compounds having formula (1) is surprising since, in contrast to epibatidine, these compounds lack the presence of the basic pyridine moiety. Also, in contrast to the above mentioned known bicyclooctanes, the present compounds are devoid of a hydroxyl group or a double bond.

Of particular interest are the compounds having formula (1) wherein R, R₁ and n have the above meanings and p has the value 1.

The compounds having formula (1) can exist both as exo and endo compounds. The invention relates to racemic mixtures and to separate isomers of compounds of formula (1).

Suitable acids with which the compounds can form pharmacologically acceptable acid addition salts are for example hydrochloric acid, sulphuric acid, phosphoric acid, nitric acid, and organic acids such as citric acid, fumaric acid, maleic acid, tartaric acid, acetic acid, methane sulphonic acid and naphthalene sulphonic acid.

The analgesic effect of the compounds according to the invention has been tested in a well known test in mice using the thermal stimulation as a noxious stimulus. The procedure of this so-called hot-plate test is similar to that described in Psychopharmacology 88, (1986), p.527-528.

The compounds were given intraperitoneally. The criterion for analgesic activity is the prolongation of latency to lick forepaws.

The compounds can be brought into forms suitable for administration by means of usual processes using auxiliary substances such as liquid and solid carrier materials.

The invention also relates to a method for the preparation of novel 2-phenyl-7-azabicycloheptane derivatives and novel 6-phenyl-8-azabicyclooctane derivatives respectively.

The compounds having formula (1) wherein p has the value 1, i.e. 2-phenyl-7-azabicycloheptane derivatives can be obtained by reduction of a compound of the formula (2) 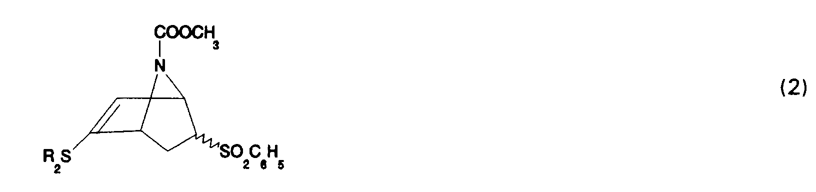
wherein R₂ is e.g. phenyl, with 6% sodium amalgam to remove the phenylsulphonyl group giving a compound of the formula (3) 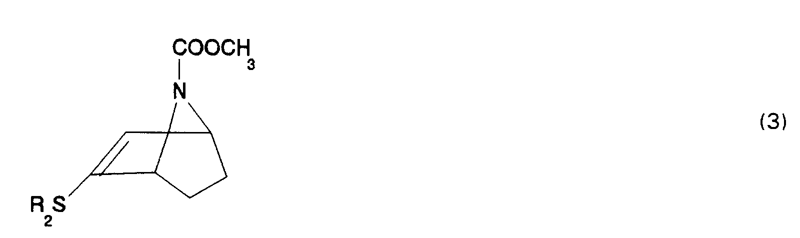
In a second step the optionally substituted phenyl group is introduced in the obtained compound having formula (3) via the palladium catalyzed vinylation reaction of e.g. bromobenzene (Heck-reaction, J.Am. 101 (1979), 4743), giving a compound of the formula (4) wherein R₁ and n have the above meanings: 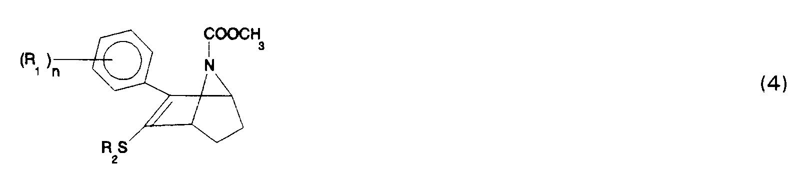
Reduction of the so-obtained compound of the formula (4) with Raney Nickel gives the endo-7-methoxycarbonyl-2-phenyl-7-azabicycloheptane derivative having formula (5) 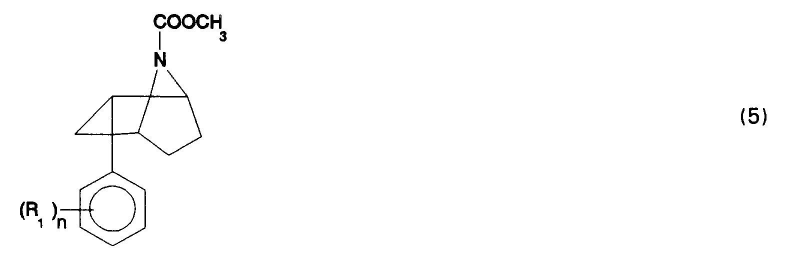
Finally the protective methoxycarbonyl group can be removed (e.g. with trimethylsilyl iodide) giving the HI-salt of a compound having formula (6) 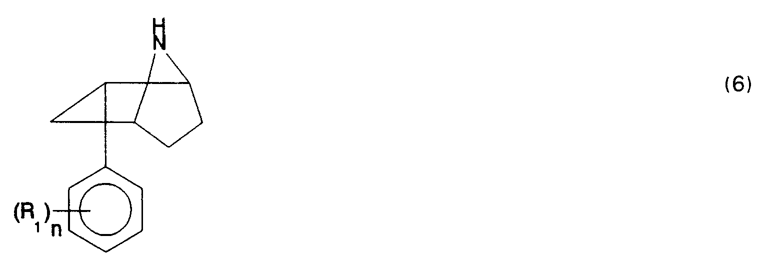
If desired the nitrogen atom in the so-obtained compound can be alkylated in a manner known per se.

The starting compound having formula (2) can be obtained as described in Heterocycles 29 (1988), 79. The compounds of formula (1) wherein p has the value 2 can be prepared by reduction of a compound (7) 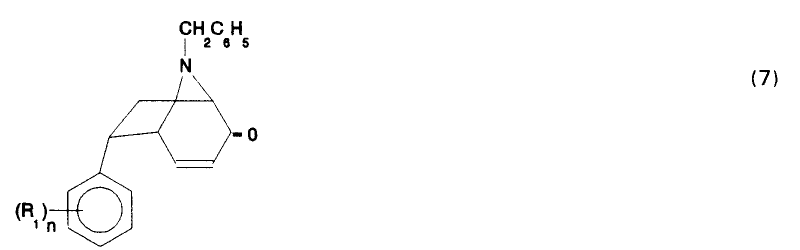
wherein R₁ and n have the above meanings, with H₂ and Pd/C. The so-obtained compound having formula (8) 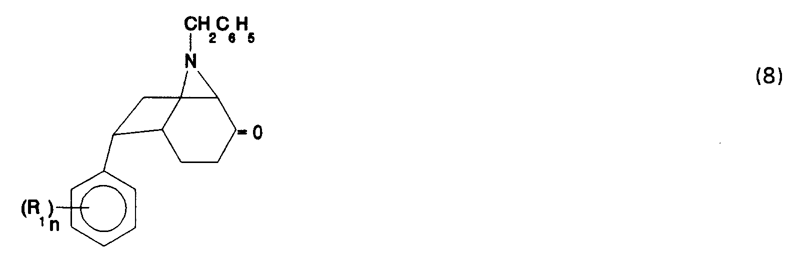
is reacted with hydrazine (NH₂NH₂.H₂0) to remove the keto-oxygen atom, giving the corresponding compound of the formula (9) 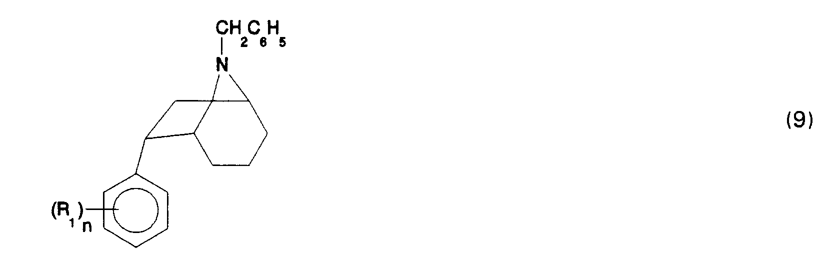
This compound (9) is then treated with hydrogen under pressure to remove the protective benzyl group giving the desired compound of formula (1) wherein R is hydrogen, R₁ and n have the above meaning and p has the value 2.
If desired the nitrogen atom in the obtained compound can be alkylated in a manner known per se.
The starting compound having formula (7) can be prepared according to the method described in Angew. Chemie, vol. 88, (1976), p. 41-49. According to this method a mixture of the endo and exo configuration is obtained.

The preparation of the compounds will now be described in the following specific examples.

### Example I: endo-2-phenyl-7-azabicyclo [2,2,1] heptane

### a) 2-phenylthio-7-methoxycarbonyl-7-azabicyclo [2,2,1] hept-2-ene

To a solution of 2.8 g (7 mmol) of 2-phenylthio-5-phenylsuphonyl-7-methoxycarbonylazabicyclo [2,2,1] hept -2-ene, and 6.1 g (42 mmol) of disodium hydrogen phosphate in 50 ml of methanol was added in three portions 16.2 g of 6% sodium amalgam. The suspension was stirred at room temperature during two hours. Subsequently the reaction mixture was quenched with water. After extraction with dichloromethane the extract was dried with Na₂SO₄ and concentrated under reduced pressure, yielding 3.15 g of crude product. Further purification was done by chromatography (n-hexane/ethyl acetate = 7/1), yielding 1.0. g of the wanted product (= 30 %).
Analytical data: High-resolution ms Calcd for C₁₄H₁₅NO₂S(M+):261.08235; found 261.08234. ¹H-NMR: δ(ppm) = 1.26 (m, 2H), 1.85 (m, 1H), 1.98 (m, 1H) 3.66 (s, 3H, OMe), 4.58 (br s, 1H, H-3), 4.81 (br s, 1H) 6.16 (br s, 1H), 7.25-7.41 (m, 5H, Ph).

### b) 2-phenylthio-3-phenyl-7-methoxycarbonyl-7-azabicyclo [2,2,1] hept-2-ene

A solution of 0.9 g (3.45 mmol) of the obtained product and 0.59 g (3.75 mmol) of bromobenzene in 1.2 g of tetramethylethylene diamine (TMEDA) was added under a nitrogen atmosphere to a solution of 155 mg (0.345 mmol) of palladium acetate, and 180 mg (0.69 mmol) of triphenylphosphine in 2 ml of dried acetonitrile. Consequently the reaction vessel is carefully sealed and kept at 95 °C for four days. The amount of conversion is monitored by GC. After cooling down the reaction mixture was quenched with water and extracted with dichloromethane. The extract was dried with Na₂SO₄ and concentrated under reduced pressure.
Further purification was done by chromatography (n-hexane/THF: 95/5), yielding 430 mg of the desired compound (yield 37%). Analytical data : High-resultion ms Calcd for C₂₀H₁₉NO₂S.(M+):337.11365 Found 337.11359. ¹H-NMR: δ(ppm) = 1.43 (m, 2H), 1.91 (m, 1H), 2.07 (m, 1H) 3,67 (s, 3H, OMe), 4.63 (br s, 1H), 5.23 (br s, 1H), 7.25-7.62 (M, 10H, Ph).

### c) endo-2-phenyl-7-methoxycarbonyl-7-azabicyclo[2,2,1]heptane

A solution of 0.6 g (1.78 mmol) of the obtained product and 6 g of Raney-Nickel in 60 ml of ethanol was refluxed during approximately four hours. The conversion of the starting material was followed by GC.
Continued reflux after 95% of the starting material had been converted resulted in a large amount of side-products. After cooling down the suspension was filtered, the residue was washed with ethanol, and the collected filtrates were concentrated under reduced pressure. Subsequently the crude product was dissolved in diethyl ether, filtered, the residue was washed with ether and the collected filtrates were concentrated under reduced pressure. Further purification was achieved by chromatography (n-hexane/ethyl acetate = 8/1), yielding 290 mgr (70%) of pure product.
Analytical data: High-resolution ms Calcd for C₁₄H₁₇NO₂ (M+): 231.12593. Found: 231.12584. ¹H-NMR: δ(ppm)= 1.43-1.58 (m, 3H), 1.67 (dd, 1H, J=12.6 Hz, J=5.9 Hz), 1.81 (dddd, 1H J= J=12 Hz, J=4 Hz, J=3 Hz), 2.25 (dddd, 1H, J=J=12 Hz, J=5 Hz, J=3Hz), 3.51 (dd, 1H,J=10 Hz,J=5,7 Hz), 3.72 (s, 3H, OMe), 4.38 (m, 2H, H-1 and H-4), 7.20-7.35 (m, 5H, phenyl).

### d) endo-2-phenyl-7-azabicyclo[2.2.1]heptane

To a solution of 0.19 g( 0.82 mmol) of the obtained compound in 20 ml of CHCl₃ was added 0.33 g (1.64 mmol) of Me₃Sil. The mixture was stirred at 60 °C during 3.5 hours. After cooling down the reaction mixture was concentrated under reduced pressure, solved in CH₂Cl₂, and consequently quenched with methanol. After refluxing for 10 minutes the mixture was concentrated under reduced pressure once more.
The crystalline product was washed with small amounts of diethyl ether, yielding 0.165 g of crude exo-2-phenyl-7-azabicyclo [2,2,1] heptane as the hydrogen iodide salt. The salt was recrystallized in acetonitrile.
Analytical data: mp 160-165 °C. High-resolution ms Calcd for C₁₂H₁₅N (M+): 173.259; found: 173.259 ¹H-NMR: δ(ppm)= 1.56-1.72 (m, 3H, (A+B) overlapping, H-3n, H-5n and H-6n), 1.74 (dd, 1H, J_{3x,2n}= 12 Hz, J_{3x,2n}=6Hz, H-2n), 1.97 (dddd, 1H, J_{5x,6x}= J_{5x,5n}=12 Hz, J_{5x,4}=4 HZ, J_{5x,6n}=3 Hz, H-5x), 2.38 (dddd, 1H, J_{6x,5x}=J_{6x,6n}=12 HZ, J_{6x,1}=5Hz, J_{6x,5n}=3 Hz, H-6x), 3.46 (br s, 1H, NH), 3.72 (dd 1H, J_{2n,3n}=10 HZ, J_{2n,3x}=6Hz, H-2n), 4.12 (m, 2H, (A=B) overlapping, H-1 and H-4), 7.20-7.37 (m, 5H, phenyl).

### Example II: 6-(3-methoxyphenyl)-8-azabicyclo[3,2,1]octane

a) An endo/exo-mixture of the compound of formula (7) wherein (R₁)ₙ is the 3-methoxy group, obtained from N-benzyl-3-hydroxypyridinium bromide and 3-methoxystyrene according to the method described in Angew. Chemie, vol. 88, (1976), p. 41-49, was separated into the exo and endo compound by means of chromatography on a neutral Al₂O₃-column with n-hexane/ethylacetate (9:1).
After evaporating, the residue is dissolved in n-hexane with a small amount of ethylacetate, and recrystallized.
Endo: Yield 3.4 g, melting point 84-85°C

| | calculated (%) | found (%) |
|---|---|---|
| C | 78.97 | 78.92 |
| N | 4.39 | 4.40 |
| H | 6.63 | 6.65 |

Exo: Yield 1.4 g, melting point 85-86°C

| | calculated (%) | found (%) |
|---|---|---|
| C | 78.97 | 78.73 |
| N | 4.39 | 4.40 |
| H | 6.63 | 6.46 |

b) The obtained endo and exo compounds are further reacted separately in the same manner . In a first step the double bond is hydrogenated with Pd/C as a catalyst. The reaction is carried out in ethanol/ethyl acetate (2 atm. H₂ for 2 hours). Pd/C is removed by filtration and the solution is evaporated, yielding the corresponding compounds of formula (8).
- Endo:: Yield 100%; purity (gas chromatography): 98%
Mcalc. 321.17288
Mfound 321.17252
- Exo:: Yield 100%; purity: 98%
Mcalc. 321.17288
Mfound 321.17220

c) The so-obtained compounds of the formula (8) (R₁ being 3-methoxy) are dissolved (12.5 mmol) in 50 ml of ethanol. To the solution 6.25 g of NH₂NH₂. H₂O (6 equivalents) is added and the mixture is heated for 3 hours on a oil bath of 120°C. Thereafter the mixture is evaporated (15 mm Hg/40°C), an excess of KOH is added, and the mixture is heated under reduced pressure on a oil bath (190-200°C). After heating for 20 minutes no further foam is observed. The mixture is cooled, water is added, and the mixture is then treated with ether and dried with Na₂SO₄. Finally the solution is evaporated by distillation.
- Endo:: yield 85%, b.p. 240°C (0.2 mm Hg), purity 97%
Mcalc. 307.19361
Mfound 307.19353
- Exo:: yield 80%; b.p. 240°C (0.2 mm Hg); purity 98%
Mcalc. 307.19361
Mfound 307.19414

d) 10 mmol of the obtained compounds having formula (9), wherein (R₁)ₙ is 3-methoxy, is dissolved in acetic acid and treated with hydrogen (20 atm.; 24 hours) to remove the benzyl group. The solution is evaporated (0.5 mm Hg), the residue is dissolved in methylene chloride, and the mixture is washed with a sodium bicarbonate solution. After drying with sodium sulfate the solution is evaporated by distillation.
Yield 2.3 g (78%) of the compound having formula 1 wherein p is 2, R is H and (R₁)ₙ is 3-methoxy; b.p. 150-160°C (0.2 mm Hg); purity 98%.

| Endo: | Exo: |
|---|---|
| Mcalc. 217.1467 | Mcalc. 217.1467 |
| Mfound 217.1463 | Mfound 217.1467 |

e) 3.25 mmol (1 g) of the compounds obtained in step c) are heated with 1.2 g (47%) of HBr (2 equivalents) during 2.5 hours on an oil bath (130°C). The mixture is cooled, 50 ml of methylene chloride and a saturated solution of sodium bicarbonate in water are added, the mixture is stirred giving a clear solution. Water is separated, the organic solution is dried with sodium sulfate and evaporated. The obtained compounds have formula (9) wherein (R₁)ₙ is 3-hydroxy.
- Endo:: yield 79%; purity 96%.
Mcalc. 293.17796
Mfound 293.17812
- Exo:: yield 78%; purity 99%
Mcalc. 293.17796
Mfound 293.17812

f) To remove the protective benzyl group the obtained compounds are dissolved in ethyl acetate/ethanol (2/1) and treated with H₂/PdC (15 atm., 45°C) for 6 hours. PdC is removed by filtration and the solution is evaporated. Ether is added to the residue and the crystalline product is obtained by filtration.
- Endo:: yield 80%; purity 99%.
Mcalc. 203.13101
Mfound 203.13096
- Exo:: yield 36%; purity 70-80%
Mcalc. 203.13101
Mfound 203.13096

| | calculated (%) | found (%) |
|---|---|---|
| C | 6.89 | 6.29 |
| N | 76.81 | 72.73 |
| H | 8.43 | 8.20 |

The so obtained compounds have formula (1) wherein p is 2, R is H, and (R₁)ₙ is 3-hydroxy.

### Example III: 6-(3,4-methylenedioxynhenyl)-8-aza-bicyclo[3,2,1]octane

a) A mixture of the endo and exo compounds of the formula (7), wherein (R₁)ₙ is the 3,4-methylenedioxy group, obtained from N-benzyl-3-hydroxypyridinium bromide and 3,4-methylenedioxystyrene, is separated as described in Example II a).
Endo: m.p. 154-156°C

| | calculated (%) | found (%) |
|---|---|---|
| N | 4.20 | 4.21 |
| C | 75.66 | 75.64 |
| H | 5.74 | 5.61 |

Exo: Mcalc. 333.13649
Mfound 333.13654
b) The endo and exo compounds are further reacted separately in the same manner. In a first step the compounds are hydrogenated (dissolved in ethylacetate/ethanol: 2/1) for 16 hours at 15 atm.
Endo: yield 100%; purity 100%; m.p. 117-118°C

| | calculated (%) | found (%) |
|---|---|---|
| N | 4.18 | 4.51 |
| C | 75.20 | 74.69 |
| H | 6.31 | 6.18 |

Mcalc 335. 15214
Mfound 335.15189
Exo: yield 100%; purity 100%
Mcalc. 335.15214
Mfound 335.15221
c) The obtained compounds of formula (8), wherein (R₁)ₙ is 3,4-methylenedioxy are reacted as described in Example IIc) with hydrazine to remove the keto oxygen atom.
Endo: yield 75%; purity 100%; m.p. 103-107°C.

| | calculated (%) | found (%) |
|---|---|---|
| N | 4.36 | 4.53 |
| C | 78.47 | 75.29 |
| H | 7.21 | 7.02 |

Mcalc. 321.17288
Mfound 321.17316
Exo: Mcalc. 321.17288
Mfound 321.17284
d) The so-obtained compounds of formula (9) wherein (R₁)ₙ is 3,4-methylenedioxy are dissolved in ethanol and treated with H₂/PdC (15 atm., 16 hours, 45°C), and purified as described in Example IId), to remove the N-benzyl group.
Endo: yield 85%; purity 100%; m.p. 174-180°C.

| | calculated (%) | found (%) |
|---|---|---|
| N | 6.06 | 5.75 |
| C | 72.70 | 65.17 |
| H | 7.41 | 6.69 |

Exo: yield 90%; purity 92%
Mcalc. 231.12593
Mfound 231.12584

## Claims

1. Compounds of the formula (1) 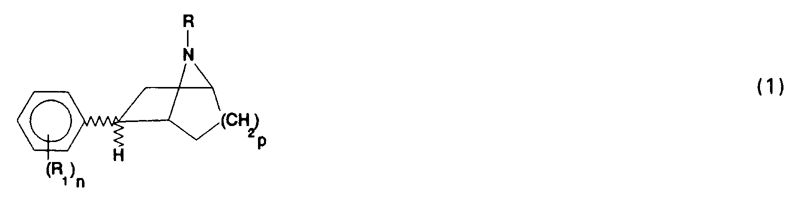 wherein
- R is hydrogen or alkyl (1-3C),
- R₁ is alkyl (1-3C), alkoxy (1-3C), halogen, hydroxy or esterified hydroxy, amino or acetylated amino, or methylenedioxy,
- n has the value 0-3, and
- p is 1 or 2,
and salts thereof with pharmacologically acceptable acids.

2. Compounds as claimed in claim 1, characterized in that p has the value 1.

3. Pharmaceutical compositions comprising at least one compound as claimed in claim 1 as an active component.

4. A method for the preparation of pharmaceutical compositions, characterized in that a compound as claimed in claim 1 is brought into a form suitable for administration.

5. A method of preparing compounds as claimed in claim 1, characterized in that a compound of formula (1) is prepared wherein R, R₁ and n have the meaning given in claim 1, and p has the value 1 by
a) reduction of a compound of formula (2) 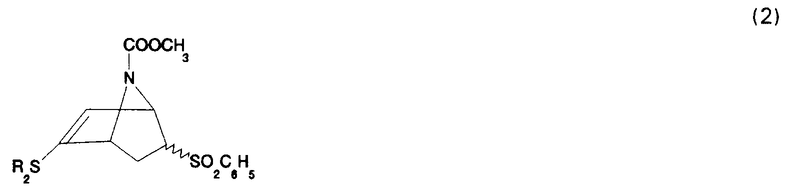 wherein R₂ is phenyl, with sodium amalgam, to remove the phenylsulphonyl group, to give the corresponding compound having formula (3) 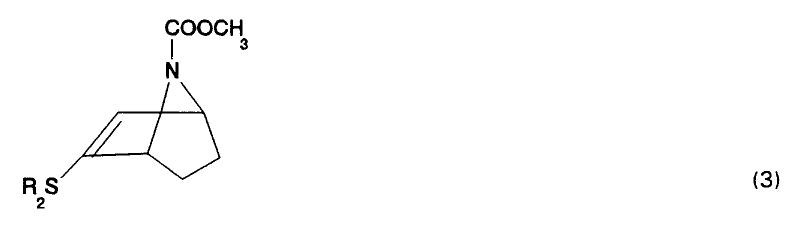
b) introduction of the 2-phenyl-(R₁)ₙ group via the Pd catalyzed vinylation reaction of bromobenzene, giving a compound having formula (4) 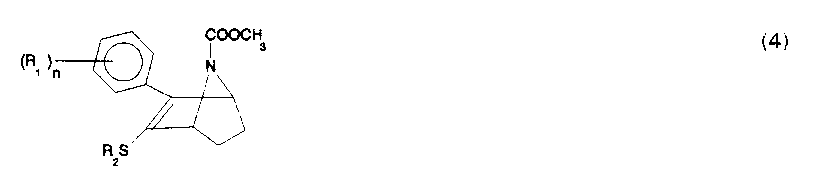
c) reduction of the double bond in the obtained compound with Raney Nickel to compound (5) 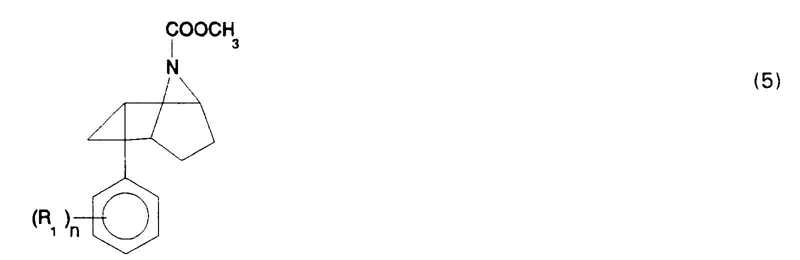
d) removing the methoxycarbonyl group from compound (5) to give compound (6)

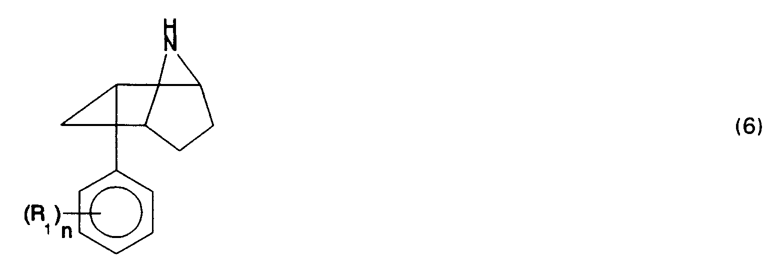

6. A method as claimed in claim 5, characterized in that a compound having formula (1) is prepared wherein R, R₁ and n have the meaning given in claim 1, and p is 2, by
a) reduction of a compound of formula (7) 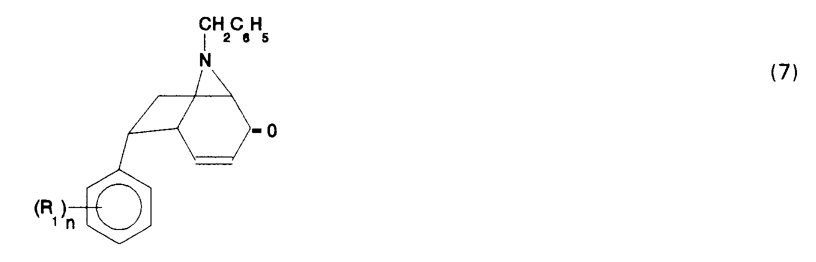 to a compound of the formula (8) 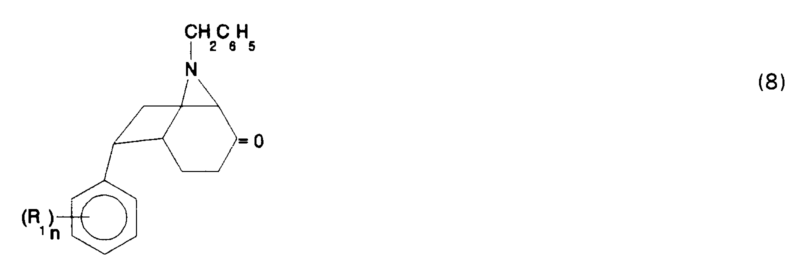
b) removing the keto oxygen atom with hydrazine to give a compound of the formula (9) 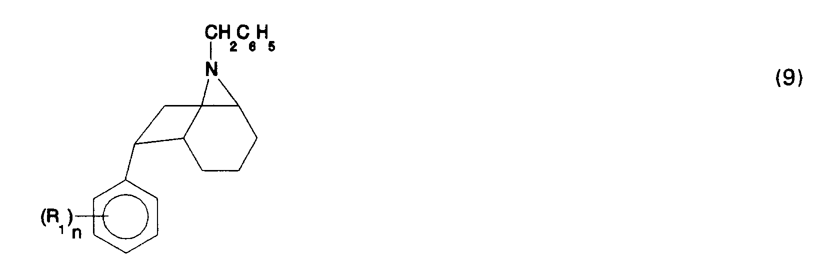 and c) removing the protective benzyl group.
